# FASCICULE DE BREVET EUROPEEN

(11) **EP 1 054 672 B1**
(45) Date de publication et mention de la délivrance du brevet: **10.01.2007**
(21) Numéro de dépôt: 99903752.6
(22) Date de dépôt: 15.02.1999
(51) Int. Cl.: A61K 31/485, A61K 9/50

(54) **MICROGRANULES DE SULFATE DE MORPHINE, PROCEDE DE FABRICATION ET PREPARATIONS PHARMACEUTIQUES**
MORPHINSULFAT ENTHALTENDE MIKROGRANULATE, VERFAHREN ZU DEREN HERSTELLUNG UND PHARMAZEUTISCHE ZUSAMMENSETZUNGEN
MORPHINE SULPHATE MICROGRANULES, METHOD FOR MAKING SAME AND PHARMACEUTICAL PREPARATIONS

(30) Priorité: 16.02.1998 FR 9801816
(43) Date de publication de la demande: 29.11.2000
(73) Titulaire: ETHYPHARM, 78550 Houdan (FR)
(72) Inventeur: DEBREGEAS, Patrice, F-75007 Paris (FR); LEDUC, Gérard, F-45330 Malesherbes (FR); OURY, Pascal, F-75005 Paris (FR); SUPLIE, Pascal, F-27400 Montaure (FR)
(74) Mandataire: Ahner, Francis
(86) Numéro de dépôt international: PCT/FR1999/000326
(87) Numéro de publication internationale: WO 1999/040918

(56) Documents cités:
- EP-A- 0 636 366
- WO-A-95/31972
- WO-A-99/20255
- US-A- 5 445 829
- US-A- 5 672 360

## Description

La présente invention concerne une nouvelle formulation du sulfate de morphine à libération immédiate, pour administration orale selon la revendication 1.

La présente invention s'étend en outre au procédé de fabrication de cette formulation et aux préparations pharmaceutiques la contenant.

Dans la présente demande, on entend par "sulfate de morphine" le sel de sulfate, éventuellement hydraté, du (5 alpha, 6 alpha)-7,8-didéhydro-4,5-époxy-17-méthylmorphinan-3,6-diol.

Dans la présente demande, on entend par "libération immédiate" la mise à disposition dans l'organisme d'un principe actif, en l'occurrence du sulfate de morphine, à une vitesse telle que la concentration plasmatique en principe actif est thérapeutiquement efficace et inférieure à la concentration toxique pendant une période de temps inférieure à environ 4 heures.

L'administration de morphine par voie orale est considérée comme le traitement de choix des douleurs chroniques.

Le document EP 655 240 décrit un comprimé à libération prolongé comprenant un noyau à libération immédiate incluant de l'acétaminophène et de la morphine qui provoque la libération de plus de 75 % de l'acétaminophène en 45 minutes lorsqu'il est placé dans 900 ml d'une solution d'acide chlorhydrique à 0,1 N.

Le document US 5 445 829 décrit une formulation contenant un mélange de particules à libération immédiate et de particules à libération retardée. Les particules à libération immédiate sont obtenues en pulvérisant une solution d'agent liant sur un mélange de principe actif et de grains inertes. Les particules à libération immédiate contiennent un liant, qui représente entre 4 et 8 % du mélange sulfate de morphine/liant.

Le document US 5 026 560 décrit des granules obtenus par pulvérisation de poudre. Ces granules sont constitués d'un noyau neutre enrobé d'un mélange de principe actif et d'hydroxypropylcellulose faiblement substituée. Le liant utilisé est une solution aqueuse d'hydroxypropylcellulose.

Le document WO 95/31972 décrit une formulation orale multiparticulaire à libération immédiate comprenant des microgranules constitués d'un noyau neutre enrobé d'un mélange de principe actif, en particulier le sulfate de morphine, et d'un diluant, comme le lactose hydraté présentant une densité apparente comprise entre 0,4 et 0,9 g/ml. Le document WO 95/31972 enseigne que la fixation du sulfate de morphine sur des noyaux neutres, nécessite l'adjonction d'un diluant pour obtenir de bons résultats.

Le document WO 96/00066 décrit des comprimés de morphine, pour administration orale, à libération contrôlée. Ces comprimés sont obtenus par compression successive de deux compositions : une première composition dite "thérapeutique" obtenue par granulation humide d'un mélange contenant du sulfate de morphine, un ou plusieurs polyoxydes d'alkylène et de la polyvinylpyrrolidone, et une deuxième composition obtenue par granulation humide d'un mélange contenant un polyoxyde d'alkylène, du chlorure de sodium et de l'hydroxypropylméthylcellulose. Le comprimé ainsi obtenu comprend une composition de principe actif enrobée d'une membrane semi-perméable poreuse qui contrôle la vitesse de libération dudit principe actif.

L'objet de la présente invention est de fournir une formulation orale de sulfate de morphine sous forme de microgranules à libération immédiate selon la revendication 1.

Les microgranules selon l'invention présentent l'avantage
- d'optimiser la distribution de l'effet thérapeutique grâce à une large distribution des granules dans le tube digestif qui contribue à une meilleure absorption du principe actif,
- d'éviter l'apparition de zones à concentration élevée en principe actif dans le tube digestif,
- de constituer une formulation stable,
- de constituer un support pour une formulation à libération prolongée ayant la même présentation galénique,
- de pouvoir être fabriqué par un procédé ne mettant en oeuvre aucun solvant organique.

Les microgranules à libération immédiate selon l'invention sont caractérisés en ce que chaque microgranule comprend un grain support neutre enrobé d'un mélange de sulfate de morphine et d'un liant pharmaceutiquement acceptable, comme l'hydroxypropylméthylcellulose, ledit liant représentant 15 à 40 % en poids, du mélange sulfate de morphine/liant.

Le grain support neutre est constitué de saccharose ou d'un mélange de saccharose et d'amidon, par exemple dans les proportions massiques 3/1. Le diamètre du grain neutre est, de préférence, compris entre 200 et 900 microns, de préférence encore entre 400 et 750 microns.

Les microgranules sont enrobés d'une couche de protection externe contenant un agent filmogène, de préférence l'hydroxypropylméthylcellulose, et un agent lubrifiant choisi parmi les lubrifiants pharmaceutiquement acceptables notamment le talc. On utilisera avantageusement l'agent lubrifiant dans une proportion telle qu'il représente 10 à 60% en poids du mélange liant/agent lubrifiant.

La couche externe représente avantageusement 1 à 5 % en poids de la masse totale des microgranules avant enrobage.

La teneur en sulfate de morphine des microgranules selon l'invention est de préférence comprise entre 50 et 200 mg/g.

La présente invention fournit des microgranules de sulfate de morphine à libération immédiate caractérisés par un profil de dissolution dans l'eau, tamponnée à un pH environ égal à 7 et à une température de 37°C par la méthode de dissolution à palette à 100 tours/min tel que :
- plus de 70% en poids de principe actif sont dissous au bout de 30 minutes,
- plus de 90% en poids de principe actif sont dissous au bout de 60 minutes.

La granulométrie moyenne des microgranules de l'invention est comprise entre 0,5 et 2 mm.

La présente invention concerne également un procédé de préparation des microgranules de sulfate de morphine à libération immédiate selon l'invention, entièrement réalisée en milieu aqueux.

Le procédé selon l'invention utilise avantageusement la méthode de montage sur grains supports neutres, en turbine perforée ou en lit d'air fluidisé, ou par toute autre technologie couramment utilisée dans l'industrie pharmaceutique pour l'obtention de microgranules.

Ledit procédé comprend une étape de montage d'une suspension aqueuse de sulfate de morphine et d'un liant sur des grains supports neutres. Cette étape consiste à préparer la suspension de montage par dissolution dans l'eau du liant, puis par mise en suspension du sulfate de morphine dans la solution obtenue, puis à pulvériser la suspension de montage sur les grains neutres.

Le liant est choisi parmi les liants pharmaceutiquement acceptables, en particulier l'hydroxypropylméthylcellulose.

Les microgranules sont éventuellement tamisés et avantageusement enrobés avec une suspension aqueuse d'un agent filmogène. La suspension est obtenue par dissolution dans l'eau d'un agent filmogène choisi parmi les agents filmogènes pharmaceutiquement acceptables et préférentiellement l'hydroxypropylméthyl-cellulose.

Il sera mis en suspension, dans cette solution d'enrobage protecteur, un lubrifiant choisi parmi les lubrifiants pharmaceutiquement acceptables, et on utilisera avantageusement du talc dans une proportion de 15 à 70 % du poids de vernis sec de l'agent filmogène utilisé.

Les microgranules protégés peuvent être lubrifiés avec du talc et/ou à nouveau tamisés avant d'être conditionnés en gélules, en blisters ou en étuis.

La présente invention a également pour objet les préparations pharmaceutiques contenant les microgranules selon l'invention, éventuellement obtenus selon le procédé décrit ci-dessus, en une quantité équivalant à une dose unitaire comprise entré 1 et 100 mg, de préférence entre 5 et 60 mg, de préférence encore entre 10 et 30 mg de sulfate de morphine.

Les préparations selon l'invention se présentent avantageusement sous forme de gélules et/ou de sachets que le patient peut soit avaler directement, soit mélanger à son repas.

Les exemples suivants illustrent l'invention sans en limiter la portée.

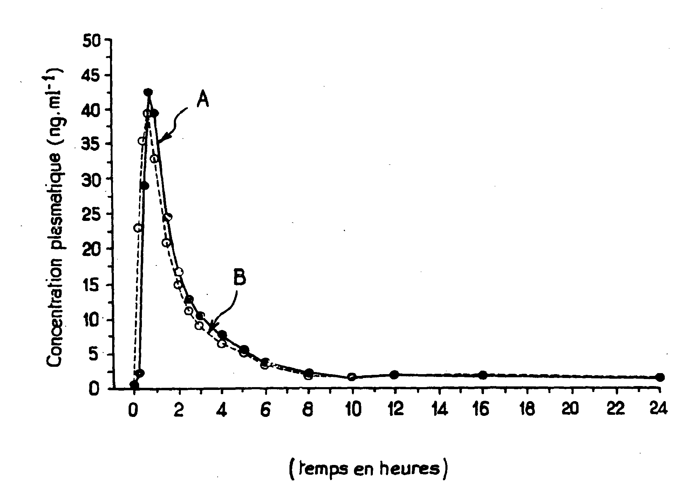

La figure unique représente l'évolution au cours du temps, de la moyenne géométrique de la concentration plasmatique en morphine mesurée chez :
- 30 patients auxquels on a administré la formulation de microgranules selon l'invention sous forme de gélules (courbe A) ;
- 30 patients auxquels on a administré une formulation buvable de chlorhydrate de morphine de l'art antérieur (courbe B).

### EXEMPLE 1 : Montage en turbine

### • Préparation de la suspension active de montage

Proportion massique des matières premières mises en oeuvre

| | |
|---|---|
| SULFATE DE MORPHINE | 18.7 % |
| PHARMACOAT 603® | 6.5 % |
| EAU PURIFIEE | 74.8 % |

La suspension est préparée dans un récipient inox. L'eau purifiée est versée dans le récipient puis mis en agitation. Le PHARMACOAT 603® (fabriqué par la société SHIN-ETSU) est incorporé par petites quantités.

L'agitation est maintenue jusqu'à complète dissolution du PHARMACOAT 603®. Le sulfate de morphine est incorporé par petites quantités.

L'agitation est maintenue jusqu'à complète homogénéité de la suspension active, puis pendant toute l'étape de montage.

### • Montage de la suspension active de sulfate de morphine sur les grains supports neutres

Des grains support NEUTRES 26® (fabriqués par la société NP-PHARM) sont placés dans une turbine perforée en rotation. Durant toute l'étape de montage, une circulation d'air chaud est maintenue au travers du lit de microgranules.
Le montage du sulfate de morphine est effectué sur les NEUTRES 26®, par pulvérisation continue de la suspension décrite ci-dessus.
La masse de microgranules est éventuellement tamisée afin de garantir l'homogénéité granulométrique du lot. A l'issue du montage, une circulation d'air non réchauffé est maintenue au travers du lit de microgranules afin de les ramener à température ambiante.

### • Préparation de la suspension d'enrobage protecteur

Proportion massique des excipients mis en oeuvre :

| | |
|---|---|
| PHARMACOAT 603® | 9.6 % |
| TALC | 4.7 % |
| EAU PURIFIEE | 85.7 % |

La suspension est préparée dans un récipient inox. L'eau purifiée est versée dans le récipient puis mise en agitation.

Le PHARMACOAT 603® est incorporé par petites quantités. L'agitation est maintenue jusqu'à complète dissolution du PHARMACOAT 603®. Le TALC est incorporé par petites quantités.

L'agitation est maintenue jusqu'à complète homogénéité de la suspension, puis pendant toute l'étape d'enrobage protecteur.
Enrobage protecteur des microgranules de sulfate de morphine

Les microgranules à protéger sont placés dans une turbine perforée en rotation. Durant toute l'étape d'enrobage protecteur, une circulation d'air chaud est maintenue au travers du lit de microgranules. L'enrobage protecteur est effectué sur les microgranules de sulfate de morphine, par pulvérisation continue de la suspension décrite ci-dessus. A l'issue de l'enrobage protecteur, une circulation d'air non réchauffé est maintenue au travers du lit de microgranules afin de les ramener à température ambiante.
La masse de microgranules est éventuellement tamisée afin de garantir l'homogénéité granulométrique du lot.

### Formule finale

| | **Quantité en %** | **Quantité en g** |
|---|---|---|
| SULFATE DE MORPHINE | 15,0 | 157,5 |
| NEUTRES 26® | 75,3 | 787,1 |
| PHARMACOAT 603® | 8,2 | 85,5 |
| TALC | 1,4 | 15,2 |
| Teneur théorique | 150 mg/g | |

### Dissolution des granules dans l'eau.

Les granules sont dissous dans 500 ml d'eau purifiée à 37°C, dans un appareil à palettes, à 100 tours/min.

La lecture des absorbances U.V. est effectuée à 285 et 310 nm.

| **Temps (en min)** | **% libéré en fonction du temps** |
|---|---|
| 5' | 61,2 |
| 10' | 88,4 |
| 15' | 91,8 |
| 20' | 93,0 |
| 25' | 93,5 |
| 30' | 93,5 |
| 35' | 93,5 |
| 40' | 93,6 |
| 45' | 93,8 |
| 50' | 93,7 |
| 55' | 93,5 |
| 60' | 93,4 |

### EXEMPLE 2 : Montage en lit d'air fluidisé

• La préparation de la suspension active de montage est effectuée comme dans l'exemple 1.
• Montage de la suspension active de sulfate de morphine sur les grains supports neutres
   Des grains support NEUTRES 26® sont placés dans un appareil à lit d'air fluidisé.
   Le montage du sulfate de morphine est effectué par pulvérisation continue de la suspension préparée précédemment sur les NEUTRES 26® fluidifiés par un courant d'air chaud. La masse de microgranules est éventuellement tamisée afin de garantir l'homogénéité granulométrique du lot.
• La préparation de la suspension d'enrobage protecteur est effectuée comme dans l'exemple 1
• Enrobage protecteur des microgranules de sulfate de morphine
   Les microgranules à protéger sont placés dans un appareil à lit d'air fluidisé.
   L'enrobage protecteur est effectué par pulvérisation continue de la suspension préparée précédemment sur les granules de sulfate de morphine fluidités par un courant d'air chaud.

A l'issue de l'enrobage protecteur, on maintiendra, les granules dans le flux d'air chaud afin de les sécher. La masse dé microgranules est éventuellement tamisée afin de garantir l'homogénéité granulométrique du lot.

### Formule finale

| | **Quantité en %** | **Quantité en g** |
|---|---|---|
| SULFATE DE MORPHINE | 14,7 | 138,3 |
| NEUTRES 26® | 75,8 | 713,3 |
| PHARMACOAT 603® | 8,0 | 75,8 |
| TALC | 1,4 | 13,6 |
| Teneur théorique | 147 mg/g | |

### Dissolution des granules dans l'eau.

On procède comme dans l'exemple 1

| **Temps (en min)** | **% libéré en fonction du temps** |
|---|---|
| 5' | 65,60 |
| 10' | 83,83 |
| 15' | 90,71 |
| 20' | 93,71 |
| 25' | 94,69 |
| 30' | 94,87 |
| 35' | 94,95 |
| 40' | 94,95 |
| 45' | 94,99 |
| 50' | 95,04 |
| 55' | 94,99 |
| 60' | 94,99 |

### EXEMPLE 3 : Montage en lit d'air fluidisé

On procède comme dans l'exemple 2 en diminuant la quantité d'enrobage protecteur.

### Formule finale

| | **Quantité en %** | **Quantité en g** |
|---|---|---|
| MORPHINE SULFATE | 15,3 | 138,3 |
| NEUTRES 26® | 77,52 | 713,3 |
| PHARMACOAT 603® | 6,73 | 61,9 |
| TALC | 0,73 | 6,7 |
| Teneur théorique | 150 mg/g | |

### Dissolution des granules et des gélules 30 mg dans l'eau.

On procède comme dans l'exemple 2

| **Temps (en min)** | **(% massique)** | **Gélules (poids total 30 mg (% massique)** |
|---|---|---|
| 5' | 67,18 | 87,84 |
| 10' | 83,57 | 97,83 |
| 15' | 90,07 | 102, 55 |
| 20' | 92,85 | 104,77 |
| 25' | 93,58 | 105,44 |
| 30' | 93,80 | 105,48 |
| 35' | 93,84 | 105,28 |
| 40' | 93,80 | 105,33 |
| 45' | 93,88 | 105,38 |
| 50' | 93,92 | 105,38 |
| 55' | 93,84 | 105,43 |
| 60' | 93,83 | 105,38 |

### EXEMPLE 4 : Montage en turbine

La préparation de la suspension active de montage et le montage sur les grains supports neutres sont réalisés comme dans l'exemple 1.

La préparation de la suspension d'enrobage protecteur et l'enrobage protecteur des microgranules de sulfate de morphine sont réalisés comme dans l'exemple 1 mais en diminuant la quantité d'enrobage protecteur.

### Formule finale

| | **Quantité en % massique** | **Quantité en kg** |
|---|---|---|
| MORPHINE SULFATE | 15,5 | 7,6 |
| NEUTRES 26® | 76,2 | 37,2 |
| PHARMACOAT 603® | 6,9 | 3,4 |
| TALC | 1,2 | 0,6 |
| Teneur théorique | 155,7 mg/g | |

### Dissolution des granules et des gélules dans l'eau.

On procède comme dans l'exemple 1

| | **Granules** | **Gélules** | | |
|---|---|---|---|---|
| | | **Poids total de 30 mg** | **Poids total de 20 mg** | **Poids total de 10 mg** |
| 5' | 72,89 % | 87,68 % | 90,44 % | 90,99 % |
| 10' | 82,66 % | 93,64 % | 96,65 % | 95,88 % |
| 15' | 88,90 % | 96,14 % | 99,06 % | 97,66 % |
| 20' | 91,93 % | 96,96 % | 99,87 % | 98,27 % |
| 25' | 93,18 % | 97,15 % | 100,06 % | 98,53 % |
| 30' | 93,48 % | 97,24 % | 100,04 % | 98,51 % |
| 35' | 93,65 % | 97,12 % | 100,02 % | 98,51 % |
| 40' | 93,67 % | 97,12 % | 100,04 % | 98,36 % |
| 45' | 93,71 % | 97,24 % | 100,02 % | 98,40 % |
| 50' | 93,87 % | 97,12 % | 100,06 % | 98,38 % |
| 55' | 93,71 % | 97,14 % | 99,94% | 98,44% |
| 60' | 93,80 % | 97,07 % | 100,06 % | 98,42 % |

### ESSAIS CLINIQUES

Une étude de bioéquivalence est réalisée avec les gélules 30 mg notées (A) par rapport à une formulation orale de chlorhydrate de morphine buvable sous la forme d'ampoules à 0,1 %, notées (B) chaque ampoule contient 10 ml de solution, soit environ 7,6 mg de morphine, tandis que chaque gélule contient 30 mg de microgranules, soit environ 22,5 mg de morphine.

L'étude est réalisée, en simple aveugle, de façon randomisée, sur 30 patients auxquels on administre A et sur 30 patients auxquels on administre B.

Les concentrations plasmatiques en morphine et en 6-glucuronide-morphine (le métabolite actif) sont mesurées par couplage chromatographie liquide haute performance spectroscopie de masse.

On observe que les paramètres pharmacocinétique obtenus avec des gélules contenant les microgranules selon l'invention (A) sont comparables à ceux obtenus avec des ampoules contenant du chlorhydrate de morphine en solution (B).

Les résultats concernant la concentration plasmatique en morphine sont résumés dans les tableaux suivants :

| **Paramètres** | **Traitement** | **Moyenne géométrique** | **Minimum / Maximum** | **Intervalle de confiance à 90 % du rapport des moyennes géométriques de A/B** | **Point optimal dans cet intervalle** |
|---|---|---|---|---|---|
| Cmax | A | 50 | 27/98 | 100 - 121 | 110 |
| (ng.ml⁻¹) | B | 45 | 20/80 | | |
| AUC_{0-∞} | A | 101 | 57/161 | 102-114 | 108 |
| (ng.ml⁻¹.h) | B | 94 | 56/172 | | |

| **Paramètre** | **Traitement** | **Moyenne des valeurs à Cmax** | **Minimum / Maximum** | **Intervalle de confiance à 90 % de la différence des moyennes de A et de B** | **Point optimal dans cet intervalle** |
|---|---|---|---|---|---|
| Tmax (h) | A | 0,75 | 0,5/1 | 0,125 - 0,250 | 0,125 |
| | B | 0,5 | 0,25/1 | | |

On constate que les points optimaux du rapport A/B pour les paramètres Cmax et AUC_{0-∞}, ainsi que leurs intervalles de confiance réciproques sont situés dans la gamme de bioéquivalence 80-125 %.

Les gélules de microgranules de la présente invention sont donc bio-équivatents -en terme de Cₘₐₓ, AUC_{o-∞} et Tₘₐₓ- à la formulation orale de chlorhydrate de morphine buvable de l'art antérieur.

Les courbes représentant l'évolution de la moyenne géométrique de la concentration plasmatique en morphine en fonction du temps, pour A et B, sont représentées sur la figure unique.

Des résultats analogues sont obtenus avec la 6-glucuronide-morphine.

Les tolérances des formulations A et B sont comparables, et aucun effet secondaire gênant n'a été observé.

## Revendications

1. Microgranules de sulphate de morphine à libération immédiate **caractérisés en ce que** :
chaque microgranule :
- comprend un grain support neutre enrobé d'un mélange homogène de sulfate de morphine et d'un liant pharmaceutiquement acceptable, ledit liant représentant 15 à 40 % en poids du mélange sulfate de morphine/liant, et
- est enrobé d'une couche de protection externe contenant un agent filmogène et un agent lubrifiant.

2. Microgranules selon la revendication 1, **caractérisés en ce que** l'agent lubrifiant représente 10 à 60% en poids du mélange liant/agent lubrifiant.

3. Microgranules selon la revendication 1 ou 2, **caractérisés en ce que** l'agent lubrifiant est le talc.

4. Microgranules selon l'une des revendications 1 à 3, **caractérisés en ce que** la couche externe représente 1 à 5 % en poids de la masse totale des microgranules avant enrobage.

5. Microgranules selon l'une des revendications précédentes, **caractérisés en ce que** le liant et/ou l'agent filmogène est l'hydroxypropylméthylcellulose.

6. Microgranules selon l'une des revendications précédentes, **caractérisés en ce que** leur teneur en sulfate de morphine est comprise entre 50 et 200 mg/g.

7. Microgranules selon l'une des revendications précédentes, **caractérisés en ce que** leur granulométrie moyenne est comprise entre 0,5 et 2 mm.

8. Procédé de préparation de microgranules selon l'une des revendications 1 à 7, **caractérisé en ce qu'**il est entièrement réalisé en milieu aqueux.

9. Procédé selon la revendication 8, **caractérisé en ce qu'**il comprend une étape de montage d'une suspension aqueuse contenant du sulfate de morphine et d'un liant, sur des grains supports neutres, en turbine ou en lit d'air fluidisé.

10. Procédé selon la revendication 9, **caractérisé en ce que** les microgranules obtenues à l'issue de l'étape de montage, sont enrobés avec une suspension aqueuse d'un agent filmogène, comme l'hydroxypropylméthylcellulose.

11. Préparations pharmaceutiques, **caractérisées en ce qu'**elles contiennent des microgranules selon l'une des revendications 1 à 7 ou obtenus par le procédé selon l'une des revendications 8 à 10, en une quantité équivalant à une dose unitaire comprise entre 1 et 100 mg de sulfate de morphine.

## Claims

1. Morphine sulfate immediate-release microgranules, **characterized in that**:
each microgranule:
- comprises a neutral support grain coated with a homogeneous mixture of morphine sulfate and of a pharmaceutically acceptable binder, said binder representing 15 to 40% by weight of the morphine sulfate/binder mixture, and
- is coated with an outer protective layer comprising a film-forming agent and a lubricating agent.

2. Microgranules according to Claim 1, **characterized in that** the lubricating agent represents 10 to 60% by weight of the binder/lubricating agent mixture.

3. Microgranules according to Claim 1 or 2, **characterized in that** the lubricating agent is talc.

4. Microgranules according to one of Claims 1 to 3, **characterized in that** the outer layer represents 1 to 5% by weight of the total mass of the microgranule before coating.

5. Microgranules according to one of the preceding claims, **characterized in that** the binder and/or the film-forming agent is hydroxypropylmethylcellulose.

6. Microgranules according to one of the preceding claims, **characterized in that** their content of morphine sulfate is between 50 and 200 mg/g.

7. Microgranules according to one of the preceding claims, **characterized in that** their mean particle size is between 0.5 and 2 mm.

8. Process for the preparation of microgranules according to one of Claims 1 to 7, **characterized in that** it is carried out entirely in aqueous medium.

9. Process according to Claim 8, **characterized in that** it comprises a stage of layering an aqueous suspension comprising morphine sulfate and a binder on neutral support grains in a pan or in a fluidized air bed.

10. Process according to Claim 9, **characterized in that** the microgranules obtained on conclusion of the layering stage are coated with an aqueous suspension of a film-forming agent, such as hydroxypropylmethylcellulose.

11. Pharmaceutical preparations, **characterized in that** they comprise microgranules according to one of Claims 1 to 7 or obtained by the process according to one of Claims 8 to 10 in an amount equivalent to a unit dose of between 1 and 100 mg of morphine sulfate.

## Patentansprüche

1. Morphinsulfat-Mikrogranalien mit sofortiger Freisetzung, **dadurch gekennzeichnet, dass**:
jede Mikrogranalie:
- ein neutrales Trägerkorn umfasst, das mit einer homogenen Mischung von Morphinsulfat und einem pharmazeutisch annehmbaren Bindemittel umhüllt ist, wobei das Bindemittel 15 bis 40 Gew.-% der Mischung Morphinsulfat/Bindemittel ausmacht, und
- von einer äußeren Schutzschicht umhüllt ist, die ein filmbildendes Mittel und ein Gleitmittel enthält.

2. Mikrogranalien nach Anspruch 1, **dadurch gekennzeichnet, dass** das Gleitmittel 10 bis 60 Gew.-% der Mischung Bindemittel/Gleitmittel ausmacht.

3. Mikrogranalien nach Anspruch 1 oder 2, **dadurch gekennzeichnet, dass** das Gleitmittel Talkum ist.

4. Mikrogranalien nach einem der Ansprüche 1 bis 3, **dadurch gekennzeichnet, dass** die äußere Schicht 1 bis 5 Gew.-% der Gesamtmasse der Mikrogranalien vor der Umhüllung ausmacht.

5. Mikrogranalien nach einem der vorangehenden Ansprüche, **dadurch gekennzeichnet, dass** das Bindemittel und/oder das filmbildende Mittel Hydroxypropylmethylcellulose ist.

6. Mikrogranalien nach einem der vorangehenden Ansprüche, **dadurch gekennzeichnet, dass** ihr Gehalt an Morphinsulfat zwischen 50 und 200 mg/g einschließlich liegt.

7. Mikrogranalien nach einem der vorangehenden Ansprüche, **dadurch gekennzeichnet, dass** ihre mittlere Teilchengröße zwischen 0,5 und 2 mm einschließlich liegt.

8. Verfahren zur Herstellung von Mikrogranalien nach einem der Ansprüche 1 bis 7, **dadurch gekennzeichnet, dass** es vollständig in wässrigem Medium durchgeführt wird.

9. Verfahren nach Anspruch 8, **dadurch gekennzeichnet, dass** es einen Schritt des Aufbringens einer wässrigen Suspension, die Morphinsulfat und Bindemittel enthält, auf neutrale Trägerkörner in einer Zentrifuge oder in einem Luft-Fließbett umfasst.

10. Verfahren nach Anspruch 9, **dadurch gekennzeichnet, dass** die am Ende des Schrittes des Aufbringens erhaltenen Mikrogranalien mit einer wässrigen Suspension eines filmbildenden Mittels, wie Hydroxypropylmethylcellulose, umhüllt werden.

11. Pharmazeutische Präparate, **dadurch gekennzeichnet, dass** sie Mikrogranalien nach einem der Ansprüche 1 bis 7 oder erhalten durch das Verfahren nach einem der Ansprüche 8 bis 10 in einer Menge enthalten, die äquivalent zu einer Dosiseinheit an Morphinsulfat zwischen 1 und 100 mg einschließlich ist.
